# EUROPÄISCHE PATENTSCHRIFT

(11) **EP 2 243 799 B1**
(45) Veröffentlichungstag und Bekanntmachung des Hinweises auf die Patenterteilung: **27.06.2012**
(21) Anmeldenummer: 10156700.6
(22) Anmeldetag: 17.03.2010
(51) Int. Cl.: C08G 77/16, C08G 77/38, C08G 77/50, A61K 8/06, A61K 8/89, C11D 1/82, C11D 3/37

(54) **Emulgator enthaltend glycerinmodifizierte Organopolysiloxane**
Emulsifier containing glycerine-modified organopolysiloxanes
Emulsifiant contenant un organopolysiloxane glycérine-modifié

(30) Priorität: 16.04.2009 DE 102009002415
(43) Veröffentlichungstag der Anmeldung: 27.10.2010
(73) Patentinhaber: Evonik Goldschmidt GmbH, 45127 Essen (DE)
(72) Erfinder: Czech, Karin, 45309, Essen (DE); Mahring, Ulrike, 45357, Essen (DE); Ferenz, Michael, 45147, Essen (DE); Hartung, Christian, 45133, Essen (DE); Fötsch, Hannelore, 45355, Essen (DE); Meyer, Jürgen, 45134, Essen (DE); Herrwerth, Sascha, 45134, Essen (DE)

(56) Entgegenhaltungen:
- EP-A1- 1 816 154
- EP-A2- 0 475 130
- US-A- 5 262 155
- US-A- 6 001 140
- US-A1- 2005 261 133
- US-A1- 2008 311 060

## Beschreibung

### Gebiet der Erfindung:

Die Erfindung betrifft Emulgatoren enthaltend glycerinmodifizierte Organopolysiloxane der allgemeinen Formel (I) sowie deren Verwendung zur Herstellung außerordentlich stabiler Emulsionen und Dispersionen.

### Stand der Technik:

Organomodifizierte Siloxane werden in den verschiedensten Applikationen eingesetzt. Ihre Eigenschaften lassen sich durch die Art der Modifikation sowie durch die Modifikationsdichte gezielt einstellen.

So können zum Beispiel mit Allylpolyethern organophile oder nichtionische hydrophile Gruppen an ein Siloxangerüst gebunden werden. Derartige Verbindungen finden ihren Einsatz zum Beispiel als Polyurethan-Schaum-Stabilisatoren und als Entschäumer in Treibstoffen.

Durch Umsetzung mit α-Olefinen wird dagegen das Siloxan mit oleophilen Gruppen verknüpft. Die so erhaltenen Siliconwachse dienen zum Beispiel als Additive in kosmetischen Anwendungen.

Es zeigt sich in vielen Anwendungsgebieten, dass die Wirkung des Siloxans entscheidend von der Verträglichkeit mit der entsprechenden Formulierung abhängt. Als Emulgatoren geeignet sind zum Beispiel Siloxane, die neben aliphatischen Gruppen auf Basis von α-Olefinen Polyether tragen, wie beispielsweise das Verkaufsprodukt ABIL^{®} EM 90 der Evonik Goldschmidt GmbH, Deutschland.

Da polyetherhaltige Verbindungen in letzter Zeit zunehmend in Kritik geraten sind, besteht Bedarf an siloxanbasierten Emulgatoren, die keine Polyethergruppen tragen, gleichzeitig aber über gute Emulgier- und Dispergiereigenschaften verfügen.

Beispielsweise können Glycerin- oder Polyglycerin-Derivate als hydrophile Komponente die Polyethergruppen ersetzen. Polyglycerin-modifizierte Siloxane werden beispielsweise in EP 1213316 beschrieben. In der Anmeldung EP 1550687 (Shin Etsu) werden glycerinmodifizierte Siloxane für die Anwendung in Emulsionen eingesetzt. Auch sind polyglycerinmodifizierte Siloxane kommerziell erhältlich, beispielsweise von Shin-Etsu unter den Namen KF-6100 oder KSG-710. EP 0 475 130 A2 beschreibt mit Glycerin und linearen Alkylresten modifizierte Organopolysiloxane.

Jedoch sind die bis dato beschriebenen glycerinmodifizierten Siloxan-Verbindungen in der Regel nicht für eine ausreichende Stabilisierung von Emulsionen und Dispersionen - wie sie beispielsweise für verkaufsfähige, kosmetische Formulierungen nötig ist - geeignet. Formulierungen müssen lagerstabil sein, um eine angemessene Haltbarkeit und konstante Qualität des Endprodukts zu gewährleisten. Hierzu müssen die Emulsionen oder Dispersionen über eine Zeit von mehreren Monaten und bei unterschiedlichen Temperaturen eine konstant stabile Zusammensetzung und Homogenität ohne Öl- oder Wasser- oder Feststoff-Separation zeigen. Speziell bei kosmetischen Emulsionspräparaten für Make-Up und Sonnenschutzanwendungen, die zusätzlich anorganische Pigmente wie zum Beispiel Titandioxid oder Eisenoxide enthalten, ist es oft schwierig, kosmetische Präparate herzustellen, die sich durch eine gute Langzeitstabilität und durch eine sehr gute Pigmentdispergierung auszeichnen. Die im Stand der Technik beschriebenen glycerinbasierten Silicon-Emulgatoren weisen hier generell eine ungenügende Emulsionsstabilisierung auf. Emulsionen, die mit Hilfe der im Stand der Technik beschriebenen glycerinhaltigen Silicon-Emulgatoren hergestellt werden, können bei Lagerung separieren und somit zu ungenügender Stabilität und nicht reproduzierbaren Ergebnissen führen.

Eine Aufgabe der vorliegenden Erfindung bestand daher darin, neuartige, reproduzierbar herstellbare, polyetherfreie, organomodifizierte Siloxane zu entwickeln, die als leistungsfähige Emulgatoren und Dispersionsmittel eingesetzt werden können und dabei eine reproduzierbar ausgezeichnete Stabilisierung der Formulierungen zeigen.

### Beschreibung der Erfindung:

Überraschenderweise wurde gefunden, dass speziell ausgewählte glycerinmodifizierte Organopolysiloxane der allgemeinen Formel (I) sehr leistungsfähige Emulgatoren und Dispersionsmittel und damit eine signifikante Verbesserung zu den bisher beschriebenen Systemen darstellen. Die hiermit hergestellten Emulsionen und insbesondere auch pigment- oder partikelhaltige Dispersionen sind reproduzierbar sehr stabil.

Die erfindungsgemäßen Emulgatoren werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Erfindung Verbindungen, wie z. B. organomodifizierte Polysiloxane, beschrieben, die verschiedene Einheiten mehrfach aufweisen können, so können diese statistisch verteilt (statistisches Oligomer) oder geordnet (Blockoligomer) in diesen Verbindungen vorkommen. Angaben zu Anzahl von Einheiten in solchen Verbindungen sind als Mittelwert, gemittelt über alle entsprechenden Verbindungen, zu verstehen.

Als Emulgator ist im Rahmen dieser Erfindung ein Emulgator zu verstehen, der mindestens aus einer Substanz der allgemeinen Formel (I) und gegebenenfalls mindestens einem Co-Emulgator besteht, wobei die Anwesenheit eines Co-Emulgators bevorzugt ist.

Gegenstand der Erfindung ist ein Emulgator enthaltend mindestens ein mit Glycerin modifiziertes Siloxan der allgemeinen Formel (I)

M_{2+f+2g-a-b}M'ₐ M"_{b} D_{c} D'_{d} D"ₑ T_{f} Q_{g} Formel (I)

mit
M = (R¹₃Si O_{1/2}),
M' = (R¹₂R² Si O_{1/2}),
M"= (R¹₂R³ Si O_{1/2}),
D = (R¹₂ Si O_{2/2}),
D' = (R¹R² Si O_{2/2}),
D" = (R¹R³ Si O_{2/2}),
T = (R¹ Si O_{3/2}) und
Q = (Si O_{4/2}),
wobei
R¹ = unabhängig voneinander gleiche oder verschiedene lineare oder verzweigte, gegebenenfalls aromatische Kohlenwasserstoffreste mit 1 bis 16 Kohlenstoffatomen, die gegebenenfalls OH- oder Esterfunktionen tragen, bevorzugt Methyl oder Phenyl, insbesondere Methyl,
R² = unabhängig voneinander gleiche oder verschiedene lineare oder verzweigte, Alkylreste mit 8 bis 28 Kohlenstoffatomen, insbesondere n-Dodecyl oder n-Hexadecyl und
R³ = -CH₂CH₂CH₂OCH₂CH(OH)CH₂OH
ist,
mit
- a =: 0 bis 2, insbesondere 0 bis 1,
- b =: 0 bis 2, insbesondere 0 bis 1,
- c =: 20 bis 300, bevorzugt 30 bis 220, insbesondere 40 bis 150,
- d =: 4 bis 130, bevorzugt 5 bis 80, insbesondere 8 bis 40,
- e =: 3 bis 75, bevorzugt 3,5 bis 45, insbesondere 4 bis 25,
- f =: 0 bis 10, bevorzugt 0 bis 5, insbesondere 0 und
- g =: 0 bis 5, bevorzugt 0 bis 2, insbesondere 0
mit der Maßgabe, dass
- N =: 2 + c + d + e + 2f + 3g = 51 bis 350, bevorzugt 60 bis 250, insbesondere 65 bis 180,
- y =: (a + d) / (b + e) = 1 bis 6, 5, bevorzugt 1,3 bis 6, insbesondere 1,5 bis 5,5 und
- z =: a + b + d + e = größer 10, bevorzugt größer 12, insbesondere größer 14
ist.

Kritisch für eine gute Performance in den Formulierungen ist speziell
a) ein langes Silicon-Backbone N,
b) mit ausreichend hoher Modifikationsdichte z und
c) einem ausgewählten Verhältnis y von hydrophoben Alkylzu hydrophilen Glycerin-Gruppen.

Dieses für eine sehr gute Stabilisierung der unten beschriebenen Emulsionen nötige Zusammenspiel wurde bisher im Stand der Technik nicht erkannt.

Bevorzugte erfindungsgemäße Emulgatoren zeichnen sich dadurch aus, dass die mit Glycerin modifizierten enthaltenen Siloxane die Maßgabe erfüllen, dass 2≥a+b>0 ist.

Für erfindungsgemäßen Emulgator kann es vorteilhaft sein, wenn die mit Glycerin modifizierten enthaltenen Siloxane weitere Verzweigungen aufweisen, um gegebenenfalls zu höheren Molekulargewichten und zu einer breiteren Molekulargewichtsverteilung zu kommen. Dies kann zu Vorteilen bei der emulsionsstabilierenden Wirkung führen.

Daher ist ein weiterer Gegenstand der vorliegenden Erfindung ein Emulgator enthaltend mit Glycerin modifizierte Siloxane der Formel (I) mit oben beschriebenen Parametern, die zusätzlich Siloxaneinheiten der allgemeinen Formel (II) enthalten.

[D"' -R⁴-D"'] Formel (II)

mit
D"'= (R¹SiO_{2/2}),
wobei
- R¹: wie in Formel (I) definiert,
- R⁴ =: -CH₂CHR¹-R⁵-CHR¹CH₂- oder -CH=CR¹-R⁵-CR¹=CH- und
- R⁵ =: ein linearer oder verzweigter Alkylenrest, der gegebenenfalls Ether-, Alkohol-, Ester-, Amin- oder Siloxan-Gruppen besitzt, oder ein Rest der allgemeinen Formel -(Si(CH₃)₂O)ₕ-Si(CH₃)₂- (mit h=1-400), z.B. -(CH₂)₄-, -(CH₂)₈-, -CH₂OCH₂-, -CH₂OCH₂C(CH₂CH₃)(CH₂OH)CH₂OCH₂-, -Si(CH₃)₂O-Si(CH₃)₂-
ist.

Es ist offenbar, dass in dem erfindungsgemäßen Emulgator Mischungen der mit Glycerin modifizierten Siloxane enthalten sein können. In diesem Zusammenhang ist es bevorzugt, dass der erfindungsgemäße Emulgator Mischungen der mit Glycerin modifizierten Siloxane mit unterschiedlichen Si-Kettenlängen, in Formel (I) als N definiert, enthält.

Die Herstellung der organomodifizierten Siloxane kann durch Hydrosilylierung erfolgen. Die zur Hydrosilylierung eingesetzten SiH-funktionellen Siloxane sind durch die dem Fachmann bekannten Verfahren der Equilibrierung, wie zum Beispiel in US 7196153 B2 beschrieben, erhältlich. Glycerinmonoallylether kann beispielweise von Raschig bezogen werden. α-Olefine wie 1-Dodecen oder 1-Hexadecen sind beispielsweise bei Shell oder Chevron Chemical erhältlich.

Die Hydrosilylierung kann nach etablierten Methoden in Gegenwart eines Katalysators durchgeführt werden. Dabei können beispielsweise Katalysatoren eingesetzt werden wie Platin-, Rhodium-, Osmium-, Ruthenium-, Palladium-, Iridium-Komplexe oder ähnliche Verbindungen bzw. die entsprechenden reinen Elemente oder deren auf Silica, Aluminiumoxid oder Aktivkohle oder ähnlichen Trägermaterialien immobilisierten Derivate. Die Hydrosilylierung kann in Gegenwart von Pt-Katalyatoren wie Cis-Platin oder Karstedt-Katalysator [Tris(divinyltetramethyldisiloxan)bis-platin] durchgeführt werden. Die eingesetzte Menge an Katalysator kann 10⁻⁷ bis 10⁻¹ mol pro mol Olefin, bevorzugt 1 bis 20 ppm, betragen. Die Hydrosilylierung kann bei Temperaturen zwischen 0 und 200 °C, bevorzugt zwischen 50 und 140 °C, durchgeführt werden. Die Reaktion kann in geeigneten Lösungsmitteln wie aliphatischen oder aromatischen Kohlenwasserstoffen, cyclischen Oligosiloxanen, Alkoholen, Carbonaten oder Estern durchgeführt werden. Durch den Einsatz eines Lösemittels läßt sich vermeiden, dass Unverträglichkeiten zwischen den Ausgangsmaterialien und damit evtl. auch im Endprodukt auftreten und dass die Initiierung der Reaktion verzögert eintritt. Es kann auch auf den Einsatz eines Lösungsmittels verzichtet werden. Geeignete Verfahren zur Hydrosilylierung werden zum Beispiel im Buch "Chemie und Technologie der Silicone", Verlag Chemie, 1960, Seite 43, sowie in US 3775452 und EP 1520870 beschrieben, auf welche ausdrücklich verwiesen wird.

Erfindungsgemäße Emulgatoren können für die Einstellung bestimmter Formulierungseigenschaften gegebenenfalls mit Co-Emulgatoren kombiniert werden. Als Co-Emulgator sind prinzipiell sämtliche Emulgatoren geeignet, die dem Fachmann bekannt sind.

Besonders bevorzugt ist die Kombination mit Co-Emulgatoren wie Polyglycerinestern der Isostearin-, Öl- und/oder Polyhydroxystearin- bzw. Polyricinolsäure, wie z.B. Polyglyceryl-4 Isostearate (z.B. ISOLAN^{®} GI 34 (Evonik Goldschmidt GmbH), Polyglyceryl-4 Diisostearate/Polyhydroxystearate/Sebacate (ISOLAN^{®} GPS (Evonik Goldschmidt GmbH), Polyglyceryl-2 Dipolyhydroxystearate oder Polyglyceryl-3 Polyricinoleate oder mit ethoxylierten Fettsäureestern der Polyhydroxystearinsäure wie PEG-30 Dipolyhydroxystearate.

Weiterhin sind bevorzugte Co-Emulgatoren Polysiloxan-Polyether-Copolymere (Dimethicone Copolyole), wie z.B. PEG/PPG-20/6 Dimethicone, PEG/PPG-20/20 Dimethicone, Bis-PEG/PPG-20/20 Dimethicone, PEG-12 oder PEG-14 Dimethicone, PEG/PPG-14/4 oder 4/12 oder 20/20 oder 18/18 oder 17/18 oder 15/15 Dimethicone, oder Polysiloxan-Alkyl-Polyether-Copolymere bzw. entsprechende Derivate, wie z.B. Lauryl oder Cetyl Dimethicone Copolyole, insbesondere Cetyl PEG/PPG-10/1 Dimethicone (ABIL^{®} EM 90 (Evonik Goldschmidt GmbH).

Ein weiterer Gegenstand der Erfindung ist die Verwendung der erfindungsgemäßen Emulgatoren zur Herstellung von Wasser-in-Öl- oder Wasser-in-Silicon-Emulsionen, die bevorzugt auch anorganische Pigmente und/oder kosmetische Partikel enthalten können.

Insbesondere wird der erfindungsgemäße Emulgator zur Herstellung kosmetischer, dermatologischer oder pharmazeutischer Formulierungen verwendet sowie zur Herstellung gegebenenfalls dispergierte Feststoffe enthaltender Pflege- und Reinigungsmittel für Haushalt oder Industrie, insbesondere für harte Oberflächen, Leder oder Textilien.

In diesem Zusammenhang sollen auch einfache Wasser-in-Öl- oder Wasser-in-Silicon-Emulsionen als Formulierungen verstanden werden.

Bevorzugt ist dabei die Verwendung der erfindungsgemäßen Emulgatoren zur Herstellung kosmetischer Emulsionen, die anorganische Pigmente (wie beispielsweise Titandioxid, Eisenoxide oder Zinkoxid) und/oder kosmetische Partikel enthalten.

Typische in der Kosmetik eingesetzte Partikel haben eine Teilchengröße von 5 - 50 µm und werden zur Erzielung eines verbesserten Hautgefühls, einer Mattierung oder zur optischen Faltenreduzierung ("soft focus effect") eingesetzt. Typische Partikelmaterialien sind PMMA, PS, PE, PP, Nylon, insbesondere Nylon-12 und Nylon-6, Siliconpartikel bzw. Siliconelastomere, Stärke, Talkum, Mica und Bornitrid. Die eingesetzten Partikel können dabei je nach Eigenschaftsprofil sowohl eine kompakte als auch poröse Struktur haben.

Besonders vorteilhaft für ein verbessertes pudriges Hautgefühl in kosmetischen Formulierungen hat sich eine Kombination der erfindungsgemäßen Emulgatoren mit Siliconelastomergelen herausgestellt. Bei den Siliconelastomergelen handelt es sich um Siliconelastomere, die direkt in einem Trägeröl hergestellt wurden. Bei den Trägerölen handelt es sich um typische kosmetische Öle, wie beispielsweise zyklische oder lineare Siliconöle, Mineralöle, Esteröle, Etheröle oder Triglyceride.

Typische Siliconelastomergele sind beispielsweise Dow Corning 9040 Silicone Elastomer Blend und Dow Corning 9041 Silicone Elastomer Blend (Dow Corning) oder die Produkte KSG-15 oder KSG-18 (Shin Etsu).

Die Kombination eines sehr guten, pudrigen Hautgefühls mit einer sogar noch verbesserten Emulgieraktivität kann dadurch erzielt werden, dass man erfindungsgemäße Emulgatoren mit alkyl- und/oder polyether- bzw. polygylcerinhaltigen Siliconelastomergelen kombiniert. Typische Siliconelastomergele mit emulgieraktiven Eigenschaften sind beispielsweise KSG-310 (PEG-15/Lauryl Dimethicone Crosspolymer; Mineral Oil) oder KSG-830 (Lauryl Dimethicone/Polyglyceryl-3 Crosspolymer; Triethylhexanoin) (Shin Etsu).

Die mit Hilfe der erfindungsgemäßen Emulgatoren hergestellten Wasser-in-Öl- und Wasser-in-Silicon-Emulsionen, insbesondere die kosmetischen, dermatologischen oder pharmazeutischen Formulierungen sowie die gegebenenfalls dispergierte Feststoffe enthaltenden Pflege- und Reinigungsmittel für Haushalt oder Industrie und die Pflege- und Reinigungsmittel für harte Oberflächen, Leder oder Textilien enthalten mindestens einen erfindungsgemäßen Emulgator und sind ebenfalls Gegenstand der Erfindung.

Bevorzugte kosmetische Formulierungen sind in diesem Zusammenhang pigmenthaltige, insbesondere anorganische Pigmente enthaltende, kosmetische Präparate wie beispielsweise solche, die Eisenoxidpigmente, Titandioxid oder Zinkoxidpartikel enthalten, und Formulierungen, die kosmetische Partikel enthalten.

Die erfindungsgemäßen Formulierungen können z.B. mindestens eine zusätzliche Komponente enthalten, ausgewählt aus der Gruppe der
Emollients,
Co-Emulgatoren und Tenside,
Verdicker/Viskositätsregler/Stabilisatoren,
UV-Lichtschutzfilter,
Antioxidantien,
Hydrotrope (oder Polyole),
Fest- und Füllstoffe,
Filmbildner,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
kosmetische Wirkstoffe,
Pflegeadditive,
Überfettungsmittel,
Lösungsmittel.

Substanzen, die als beispielhafte Vertreter der einzelnen Gruppen eingesetzt werden können, sind dem Fachmann bekannt und können beispielsweise der deutschen Anmeldung DE 102008001788.4 entnommen werden.

Da erfindungsgemäßer Emulgator hohe Leistung erzielt, ohne auf Polyetherstrukturen zurückgreifen zu müssen, sind bevorzugte erfindungsgemäße Formulierungen **dadurch gekennzeichnet, dass** die Formulierung im Wesentlichen frei von Polyethern und Polyether enthaltenden Verbindungen ist. Der Begriff "im Wesentlichen frei von Polyethern und Polyether enthaltenden Verbindungen" beschreibt im Zusammenhang mit der vorliegenden Erfindung, dass eingesetzte Verbindungen nur in Spuren, bevorzugt keine, Alkoxygruppen, Oligoalkoxygruppen oder Polyalkoxygruppen wie z.B. Ethylenoxid oder Propylenoxid enthalten. Die Konzentration an Polyether enthaltenden Verbindungen sollte kleiner 0,1 Gew.-%, insbesondere bevorzugt kleiner 0,01 Gew.-%, bezogen auf die Gesamtformulierung, bevorzugt unterhalb der Nachweisgrenze gängiger Analyseverfahren wie beispielsweise NMR-Spektroskopie, GPC oder MALDI sein.

Bevorzugt ist die Verwendung erfindungsgemäßer Emulgatoren zur Herstellung kosmetischer oder pharmazeutischer Formulierungen. Solche Formulierungen können beispielsweise Cremes, Lotionen oder Sprays, wie etwa Pflegecremes, Babycremes oder Sonnenschutzlotionen, Salben, Antiperspirantien, Deodorantien oder Schminken sein. Insbesondere kann es sich bei den kosmetischen Formulierungen auch um Formulierungen wie Make-Ups oder Sonnenschutzprodukte handeln, die dispergierte Feststoffe wie beispielsweise Eisenoxidpigmente, Titandioxid oder Zinkoxidpartikel enthalten.

Erfindungsgemäße Formulierungen können daher Verwendung als ein Hautpflege-, Gesichtspflege-, Kopfpflege-, Körperpflege-, Intimpflege-, Fußpflege-, Haarpflege-, Nagelpflege-, Zahnpflege- oder Mundpflegeprodukt finden.

Erfindungsgemäße Formulierungen können Verwendung in Form einer Emulsion, einer Suspension, einer Lösung, einer Creme, einer Salbe, einer Paste, eines Gels, eines Öls, eines Puders, eines Aerosols, eines Stiftes, eines Sprays, eines Reinigungsproduktes, eines Schmink- oder Sonnenschutzpräparates oder eines Gesichtswassers finden.

In den nachfolgend aufgeführten Beispielen wird die vorliegende Erfindung beispielhaft beschrieben, ohne dass die Erfindung und deren Anwendungsbreite auf die in den Beispielen genannten Ausführungsformen beschränkt sein sollen.

### Beispiele:

Allgemeine Arbeitsvorschrift zur Herstellung der Alkyl/Glycerin-modifizierten Siloxane nach Formel (I):

Mz_{+f+2g-a-b} M'ₐ M"_{b} D_{c} D'_{d} D"ₑ T_{f} Q_{g} Formel (I)

mit
M = (R¹₃Si O_{1/2}),
M' = (R¹₂R² Si O_{1/2}),
M" = (R¹₂R³ Si O_{1/2}),
D = (R¹₂ Si O_{2/2}),
D' = (R¹R² Si O_{2/2}),
D" = (R¹R³ Si O_{2/2}),
T = (R¹ Si O_{3/2}) und
Q = (Si O_{4/2}),
wobei für die vorliegenden Beispiele gilt:
R¹ = Methyl,
R² = Alkyl,
R³= -CH₂CH₂CH₂OCH₂CH(OH)CH₂OH und
f = g = 0.

In einem Vierhalskolben, versehen mit Rührer, Tropftrichter, Thermometer und Rückflusskühler wurden 1,3·(a+d) mol α-Olefin, 1,3·(b+e) mol Glycerinmonoallylether und 10 ppm Karstedt-Katalysator (bezogen auf die Rohstoffe ohne Lösungsmittel) in ca. 30% Toluol (bezogen auf die Gesamteinwaage) vorgelegt und auf 95°C erhitzt. 1 mol des SiH-Siloxans der allgemeinen Formel (III)

[Me₃SiO_{1/2}]_{2-a-b}[Me₂HSiO_{1/2} ]_{a+b}[Me₂SiO_{2/2}]_{c}[MeHSiO_{2/2}] ]_{d+e} Formel (III)

wurde zugetropft und der Ansatz für 2 h bei 95 °C gerührt. Laut SiH-Wert-Bestimmung wurde ein vollständiger Umsatz des SiH-Siloxans erhalten. Flüchtige Anteile wurden anschließend im Vakuum bei ca. 1 mbar und 110 °C abdestilliert. Es wurde jeweils ein viskoses, leicht trübes, fast farbloses Produkt erhalten.

In Tabelle 1 sind die nach dieser allgemeinen Vorschrift hergestellten Siloxane aufgeführt.

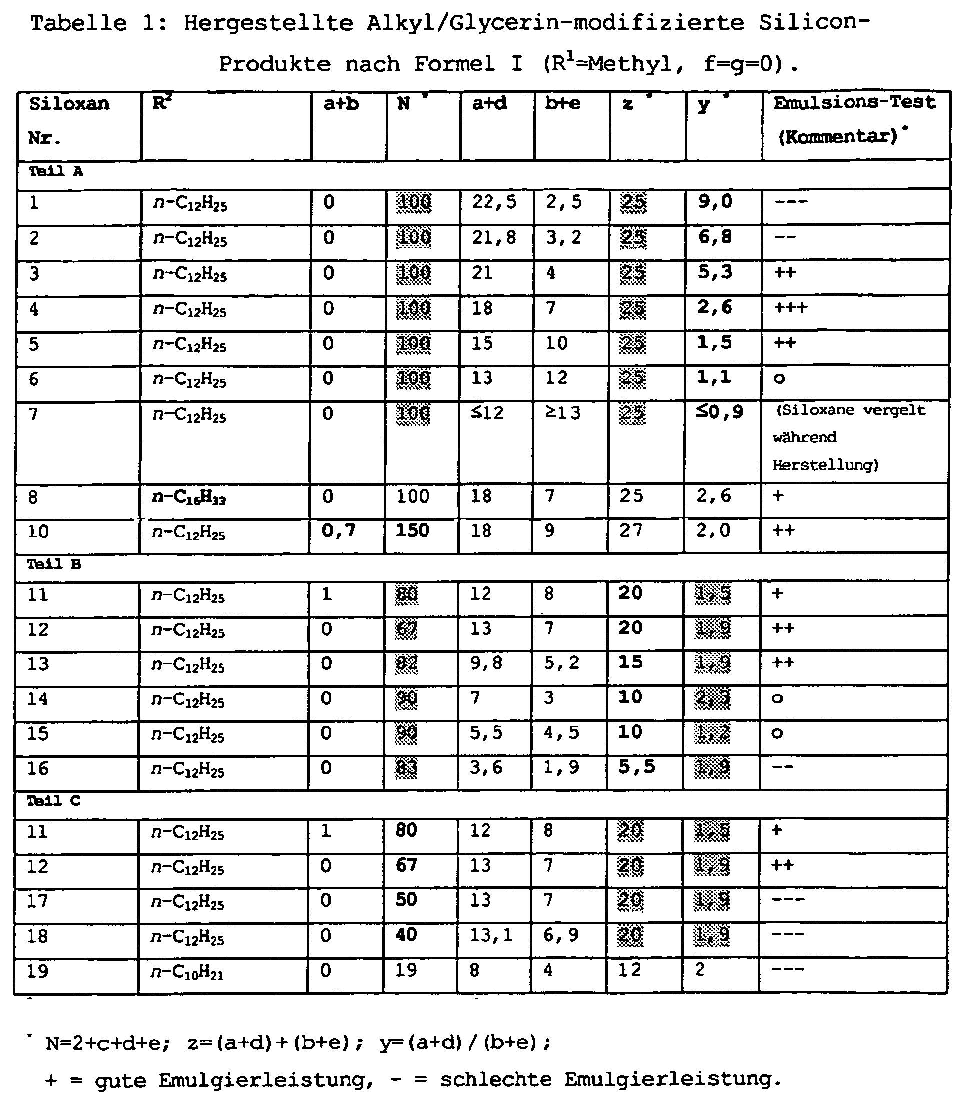

Zudem ist in der letzten Spalte der Tabelle 1 die Emulgierleistung der Produkte in einer sehr kritischen Kosmetikformulierung zusammengefasst, die eine schnelle Differenzierung der Emulsionsstabilisierung bereits nach 20 Stunden ermöglicht. Diesem kritischen Emulsionstest liegt folgende Formulierung zugrunde:

| | | |
|---|---|---|
| A | Alkyl/Glycerin-modifizierte Silicon-emulgatorsysteme (aus Tab. 1) | 1,2 % |
| | Ethylhexyl Palmitate | 11,9 % |
| | Caprylic/Capric Triglyceride | 11,9 % |
| B | Sodium Chloride | 0,8 % |
| | Glycerin | 3,0 % |
| | Water (demineralized) | 65,2 % |
| c | Phenonip^{®} XB (Clariant) (Phenoxyethanol; Methylparaben; Propylparaben; Ethylparaben) | 1,0 % |
| D | Ethanol | 5,0 % |

| | | |
|---|---|---|
| Herstellung: Langsame Zugabe von Phase B zu A unter Rühren bei Raumtemperatur; kurz homogenisieren; Zugabe von Phase C und D; homogenisieren. | | |

Die kritischen Testemulsionen wurden 20 h bei 60 °C, 50 °C sowie Raumtemperatur gelagert. Die Viskositäten der Emulsionen nach Herstellung und nach 20 h 50 °C-Lagerung wurden gemessen. Weiterhin wurden die Emulsionen einmal über Nacht bei -15 °C eingefroren und anschließend wieder aufgetaut und bewertet. Eine hervorragende (+++) Emulsionsstabilität bezeichnet eine Emulsion, die in diesem Test keinerlei Anzeichen von Wasser- oder Ölseparation bei allen Lagertemperaturen zeigte und die außerdem keinen Viskositätsabfall aufwies. Eine sehr gute (++) Emulsionsstabilität zeigt maximal eine Spur Wasserabscheidung bei 60 °C-Lagerung. Eine gute (+) Emulsionsstabilität darf darüber hinaus auch Schwächen in der Gefrierstabilität zeigen. Eine ordentliche (O) Emulsionsstabilität darf eine deutlichere Wasserabscheidung bei 60 °C, sowie Spuren Wasserabscheidung bei 50 °C zeigen und Schwächen in der Gefrierstabilität zeigen. Eine schwache (-) Emulsionsstabilität weist deutliche Anzeichen von Wasserseparation bei allen Lagertemperaturen auf. Eine sehr schwache (--) Emulsionsstabilität beschreibt größere Wasserabscheidungen bei allen Lagertemperaturen oder deutlichen Viskositätseinbruch der Testemulsion bei gleichzeitig signifikanter Wasserseparation bei Raumtemperatur. Eine katastrophale (---) Emulsionsstabilität bedeutet, dass entweder gar keine W/O Emulsion entsteht oder dass diese sich direkt nach Herstellung auftrennt.

Somit ergaben sich folgende Ergebnisse für die Alkyl/Glycerinmodifizierten Polysiloxane aus Tabelle 1: Beispiele 1-7 zeigen, dass bei konstanter und hier hoch gewählter Siliconkettenlänge (N=100) sowie konstantem und ebenfalls hoch gewähltem Modifizierungsgrad (z=25) das Verhältnis y von hydrophoben Alkyl- zu hydrophilen Glycerin-Gruppen einen entscheidenden Einfluß auf die Emulsionsstabilität hat. Bei hohen Glycerinanteilen (Beispiel 7) führt die Synthese zu unbrauchbaren Produkten, da aufgrund der hohen Hydroxylgruppen-Anzahl die Produkte über SiOC-Bildung vernetzen können und damit meist vergelen. Gute Emulsionsstabilität wird bei y = ca. 1,5 bis 5,5 beobachtet (Beispiele 3-5). Unterhalb (Beispiel 6) oder oberhalb (Beispiele 1-2) dieser Werte wird das Hydrophil-zu-Hydrophob-Verhältnis ungünstig für eine ausreichende Stabilisierung. Es müssen sowohl genügend hydrophile Glyceringruppen als auch ausreichend viele hydrophobe Alkylgruppen für eine gute Emulsionsstabilisierung vorhanden sein. Beispiel 8 (Hexadecyl- Gruppen) sowie Beispiel 10 (zusätzliche α,ω-Modifizierung) weisen ähnliche Parameter wie Beispiele 3-5 auf und zeigen entsprechend ebenfalls gute Emulsionsstabilisierung.

Bei den Beispielen 11-16 ist nun die Siliconkettenlänge fast konstant und hoch gewählt (N=67-90) sowie das Verhältnis von hydrophoben Alkyl- zu hydrophilen Glycerin-Gruppen fast konstant auf einen aus den ersten Beispielen ermittelten guten Wert (y=1,5-2,3) eingestellt worden. Der hier variierte Parameter Modifizierungsgrad z muß >10 liegen, damit gute Ergebnisse in der Emulsionsstabilisierung resultieren.

Schließlich wurde in Teil C der Tabelle 1 das Verhältnis von hydrophoben Alkyl- zu hydrophilen Glycerin-Gruppen (y=1,5-1,9) und der Modifizierungsgrad (z=20) fast konstant auf guten Werten gehalten und die Siliconkettenlänge variiert. Man erkennt deutlich, dass eine Siliconkettenlänge von N>50 für eine gute Emulsionsstabilisierung notwendig ist.

Zusammenfassend wurde festgestellt, dass für eine gute Emulgier-Performance in den Formulierungen ein Alkyl/Glycerinmodifizierter Siloxanemulgator mit
a) einem langen Silicon-Backbone (N>50),
b) ausreichend hoher Modifikationsdichte (z>10) und
c) einem ausgewählten Verhältnis von hydrophoben Alkyl- zu hydrophilen Glycerin-Gruppen (zumindest y=1-6,5)
notwendig ist.

### Anwendungsbeispiele:

Die beschriebenen kosmetischen Emulsionen sollen dazu dienen, die Verwendbarkeit der Alkyl/Glycerin-modifizierten Organopolysiloxane als Emulgatoren für kosmetische Emulsionen beispielhaft zu illustrieren.

Die Herstellung erfolgte jeweils durch Einbringen der Wasserphase in die Ölphase und anschließendes Homogenisieren nach üblichen Methoden.

Nomenklatur gemäß INCI:
W/O Creme:

| | **1** | **2** | **3** |
|---|---|---|---|
| Polysiloxan Bsp.4 | 2,0 % | | |
| Polysiloxan Bsp.10 | | 2,0 % | |
| Polysiloxan Bsp.12 | | | 2,0 % |
| Hydrogenated Castor Oil | 0,1 % | 0,1 % | 0,1 % |
| Microcrystalline Wax | 0,1 % | 0,1 % | 0,1 % |
| Isopropyl Palmitate | 8,2 % | 8,2 % | 8,2 % |
| Ethylhexyl Palmitate | 9,6 % | 9,6 % | 9,6 % |
| Sodium Chloride | 0,8 % | 0,8 % | 0,8 % |
| Glycerin | 3,0 % | 3,0 % | 3,0 % |
| Water | ad 100 % | ad 100 % | ad 100 % |
| 2-Bromo-2-Nitro-propan-1,3-Diol | 0,05 % | 0,05 % | 0,05 % |

W/Si Lotion:

| | **4** | **5** | **6** |
|---|---|---|---|
| Polysiloxan Bsp.4 | 2,0 % | | |
| Polysiloxan Bsp.10 | | 2,0 % | |
| Polysiloxan Bsp.12 | | | 2,0 % |
| Cyclopentasiloxane | 20,8 % | 20,8 % | 20,8 % |
| Sodium Chloride | 0,5 % | 0,5 % | 0,5 % |
| Glycerin | 3,0 % | 3,0 % | 3,0 % |
| Water | ad 100 % | ad 100 % | ad 100 % |
| 2-Bromo-2-Nitro-propan-1,3-Diol | 0,05 % | 0,05 % | 0,05 % |

W/O Body Lotion:

| | **7** | **8** | **9** |
|---|---|---|---|
| Polysiloxan Bsp.4 | 3,0 % | | |
| Polysiloxan Bsp.10 | | 3,0 % | 1,0 % |
| Polysiloxan Bsp.12 | | | 2,0 % |
| Hydrogenated Castor Oil | 0,25 % | 0,25 % | 0,25 % |
| Microcrystalline Wax | 0,25 % | 0,25 % | 0,25 % |
| Ethylhexyl Palmitate | 8,5 % | 8,5 % | 8,5 % |
| Caprylic/Capric Triglyceride | 8,0 % | 8,0 % | 8,0 % |
| Isopropyl Palmitate | 8,0 % | 8,0 % | 8,0 % |
| Sodium Chloride | 0,8 % | 0,8 % | 0,8 % |
| Glycerin | 3,0 % | 3,0 % | 3,0 % |
| Water | ad 100 % | ad 100 % | ad 100 % |
| Phenonip® XB (Clariant) (Phenoxyethanol; Methylparaben; Propylparaben; Ethylparaben) | 0, 7 % | 0, 7 % | 0, 7 % |

W/O Foundation (Make-Up):

| | **10** | **11** | **12** | **13** | **14** |
|---|---|---|---|---|---|
| Polysiloxan Bsp.4 | 3,0 % | 2,0 % | 2,0 % | 2,0 % | 2,0 % |
| Polyglyceryl-4 Diiso-stearate/Polyhydroxystearate/Sebacate ¹⁾ | | 1,0 % | | | |
| PEG-30 Dipoly-hydroxystearate ²⁾ | | | 1,0 % | | |
| Cetyl PEG/PPG-10/1 Dimethicone ³⁾ | | | | 1,0 % | |
| Bis-PEG/PPG-14/14 Dimethicone; Cyclo-pentasiloxane ⁴⁾ | | | | | 1,0 % |
| Diethylhexyl Carbonate | 9,8 % | 9, 8 % | 9, 8 % | 9, 8 % | 9, 8 % |
| Isohexadecane | 6, 7 % | 6, 7 % | 6, 7 % | 6, 7 % | 6, 7 % |
| Dimethicone | 1,5 % | 1,5 % | 1,5 % | 1,5 % | 1,5 % |
| Titanium Dioxide, Alumina, Triethoxy-caprylylsilane ⁵⁾ | 8,0 % | 8,0 % | 8,0 % | 8,0 % | 8,0 % |
| Iron Oxide ⁶⁾ | 1, 8 % | 1,8 % | 1,8 % | 1,8 % | 1,8 % |
| Disteardimonium Hectorite | 0,2 % | 0,2 % | 0,2 % | 0,2 % | 0,2 % |
| Aluminum Starch Octenyl Succinate | 1,5 % | 1,5 % | 1,5 % | 1,5 % | 1,5 % |
| Nylon-12 | 1,5 % | 1,5 % | 1,5 % | 1,5 % | 1,5 % |
| Glycerin | 2,0 % | 2,0 % | 2,0 % | 2,0 % | 2,0 % |
| Sodium Chloride | 0,8 % | 0,8 % | 0,8 % | 0,8 % | 0,8 % |
| Water | ad 100 % | ad 100 % | ad 100 % | ad 100 % | ad 100 % |
| Phenonip^{®} XB (Clariant) (Phenoxyethanol; Methylparaben; Propyl-paraben; Ethylparaben) | 0,8 % | 0,8 % | 0,8 % | 0,8 % | 0,8 % |

| | | | | | |
|---|---|---|---|---|---|
| ¹⁾ ISOLAN^{®} GPS (Evonik Goldschmidt GmbH) ²⁾ ARLACEL^{®} P-135 (Croda) ³⁾ ABIL^{®} EM 90 (Evonik Goldschmidt GmbH) ⁴⁾ ABIL^{®} EM 97 (Evonik Goldschmidt GmbH) ⁵⁾ AC 360 (Kemira) ⁶⁾ SICOVIT^{®} Brown 70E172 (BASF) | | | | | |

Zum Einsatz erfindungsgemäßer Alkyl-Glyceryl-Polysiloxane analog Beispielformulierung 10 ist zu sagen, dass diese meist zu besseren Stabilitäten und höheren Farbintensitäten führen, als die gängigen Emulgatoren aus dem Stand der Technik (z.B. ISOLAN^{®} GPS, ARLACEL^{®} P-135, ABIL^{®} EM 90, ABIL^{®} EM 97).

Gleichwohl kann im Einzelfall, etwa aus Gründen der Viskositätsregulierung oder zur Einstellung eines spezifischen Hautgefühls, eine Kombination mit Emulgatoren aus dem Stand der Technik vorteilhaft sein. Dies ist in der Regel leicht möglich (Beispielformulierungen 11-14). Auch die Verwendung einer Kombination zweier erfindungsgemäßer Emulgatoren mit unterschiedlichen Silicon-Kettenlängen kann besonders vorteilhaft für die Emulsionsstabilisierung sein (Beispielformulierung 9).

Neben dem Einsatz gecoateter Titaniumdioxide (z.B. AC 360) sind die erfindungsgemäßen Polysiloxan-Emulgatoren auch in der Lage, nichtgecoatete Pigmente zu stabilisieren. Auch hier werden in der Regel höhere Stabilität und höhere Farbintensitäten erhalten als bei Emulgatoren aus dem Stand der Technik.

### W/O - Antiperspirant Roll-on:

| | **15** | **16** | **17** |
|---|---|---|---|
| Polysiloxan Bsp.4 | 3,0 % | | 2,0 % |
| Polysiloxan Bsp.8 | | 3,0 % | |
| Bis-PEG/PPG-14/14 Dimethicone; Cyclo-pentasiloxane ⁴⁾ | | | 1,0 % |
| Cyclopentasiloxane | 20,0 % | 20,0 % | 20,0 % |
| C12-15 Alkyl Benzoate | 4,0 % | 4,0 % | 4,0 % |
| Silica Silylate ⁷⁾ | 0,2 % | 0,2 % | 0,2 % |
| Aluminium Chlorhydrate | 20,0 % | 20,0 % | 20,0 % |
| Water | ad 100 % | ad 100 % | ad 100 % |
| Preservative, Parfum | q.s. | q.s. | q.s. |

| | | | |
|---|---|---|---|
| ⁴⁾ABIL^{®} EM 97 (Evonik Goldschmidt GmbH) ⁷⁾AEROSIL^{®} R 812 (Evonik Degussa) | | | |

### W/O - Sonnenschutzformulierung

| | **18** |
|---|---|
| Polysiloxan Bsp.4 | 3,0 % |
| Cetyl Dimethicone | 0,5 % |
| Diethylhexyl Carbonate | 11,5 % |
| C12-15 Alkyl Benzoate | 6,0 % |
| Ethylhexyl Methoxycinnamate | 7,5 % |
| Isoamyl p-Methoxycinnamate | 7,5 % |
| Ethylhexyl Triazone | 1,0 % |
| Bis-Ethylhexyloxyphenol Methoxyphenyl Triazine | 2,5 % |
| Titanium Dioxide; Trimethoxy-caprylylsilane ⁸⁾ | 4,0 % |
| Cyclopentasiloxane; Dimethicone Crosspolymer ⁹⁾ | 3,0 % |
| Hydrogenated Castor Oil | 0,5 % |
| Microcrystalline Wax | 0,5 % |
| Glycerin | 3,0 % |
| Sodium Chloride | 0,8 % |
| Creatine | 0,2 % |
| Water | ad 100 % |
| Preservative, Parfum | q.s. |

| | |
|---|---|
| ⁸⁾ TEGO^{®} Sun T 805 (Evonik Goldschmidt GmbH) ⁹⁾ Dow Corning 9040 Silicone Elastomer Blend (Dow Corning) | |

### W/O - Make-Up

| | **19** |
|---|---|
| Polysiloxan Bsp.8 | 4,5 % |
| Cetyl Dimethicone | 1,0 % |
| Diethylhexyl Carbonate | 4,5 % |
| Ethylhexyl Palmitate | 1,5 % |
| Dimethicone | 5,0 % |
| Cyclopentasiloxane | 9,0 % |
| Phenyl Trimethicone | 1,0 % |
| Lauryl Dimethicone/Polygly-ceryl-3 Crosspolymer; Triethylhexanoin ¹⁰⁾ | 2,0 % |
| Nylon-12 | 1,0 % |
| Iron Oxides | 2,0 % |
| Titanium Oxide | 6,0 % |
| Zinc Oxide | 0,5 % |
| Glycerin | 3,0 % |
| Sodium Chloride | 0,8 % |
| Creatine | 0,2 % |
| Water | ad 100 % |
| Preservative, Parfum | q.s. |

| | |
|---|---|
| ¹⁰⁾ KSG-830 (Shin Etsu) | |

## Patentansprüche

1. Emulgator enthaltend mindestens ein mit Glycerin modifiziertes Siloxan der allgemeinen Formel (I)
M_{2+f+2g-a-b} M'ₐ M"_{b} D_{c} D'_{d} D" ₑ T_{f} Q_{g} Formel (I)
mit
M = (R¹₃ Si O_{1/2}),
M' = (R¹₂R² Si O_{1/2}),
M" = (R¹₂R³ Si O_{1/2}),
D = (R¹₂ Si O_{2/2}),
D' = (R¹R² Si O_{2/2}),
D" = (R¹R³ Si O_{2/2}),
T = (R¹ Si O_{3/2}) und
Q = (Si O_{4/2}),
wobei
R¹ = unabhängig voneinander gleiche oder verschiedene lineare oder verzweigte, gegebenenfalls aromatische Kohlenwasserstoffreste mit 1 bis 16 Kohlenstoffatomen, die gegebenenfalls OH- oder Esterfunktionen tragen,
R² = unabhängig voneinander gleiche oder verschiedene lineare oder verzweigte Alkylreste mit 8 bis 28 Kohlenstoffatomen und
R³ = -CH₂CH₂CH₂OCH₂CH(OH)CH₂OH
ist,
mit
a = 0 bis 2,
b = 0 bis 2,
c = 20 bis 300,
d = 4 bis 130,
e = 3 bis 75,
f = 0 bis 10 und
g = 0 bis 5
mit der Maßgabe, dass
N = 2 + c + d + e + 2f + 3g = 51 bis 350,
y = (a + d) / (b + e) = 1 bis 6,5 und
z = a + b + d + e = größer 10
ist.

2. Emulgator gemäß Anspruch 1, **dadurch gekennzeichnet, dass** 2≥a+b>0.

3. Emulgator gemäß Anspruch 1 oder 2, **dadurch gekennzeichnet, dass** die mit Glycerin modifizierten Siloxane zusätzliche Siloxaneinheiten der allgemeinen Formel (II)
[D"'-R⁴-D"'] Formel (II)
mit
D"'= (R¹SiO_{2/2}),
wobei
R⁴ = -CH₂CHR¹-R⁵-CHR¹CH₂- oder -CH=CR¹-R⁵-CR¹=CH- und
R⁵ = ein linearer oder verzweigter Alkylenrest, der gegebenenfalls Ether-, Alkohol-, Ester-, Amin- oder Siloxan-Gruppen besitzt, oder ein Rest der allgemeinen Formel - (Si(CH₃)₂O)ₕ-Si(CH₃)₂-, mit h=1- 400,
ist,
enthalten.

4. Emulgator gemäß mindestens einem der Ansprüche 1 bis 3, **dadurch gekennzeichnet, dass** Mischungen der mit Glycerin modifizierten Siloxane mit unterschiedlichen Si-Kettenlängen enthalten sind.

5. Verwendung mindestens eines Emulgators gemäß mindestens einem der Ansprüche 1 bis 4 zur Herstellung von Wasser-in-Öl- oder Wasser-in-Silicon-Emulsionen sowie zur Herstellung kosmetischer, dermatologischer oder pharmazeutischer Formulierungen.

6. Verwendung gemäß Anspruch 5 zur Herstellung kosmetischer Emulsionen, die anorganische Pigmente oder kosmetische Partikel enthalten.

7. Verwendung gemäß Anspruch 5 zur Herstellung gegebenenfalls dispergierte Feststoffe enthaltender Pflege- und Reinigungsmittel für Haushalt oder Industrie, insbesondere für harte Oberflächen, Leder oder Textilien.

8. Wasser-in-Öl-, Wasser-in-Silicon-Emulsionen und Dispersionen enthaltend mindestens einen Emulgator gemäß mindestens einem der Ansprüche 1 bis 4.

9. Kosmetische, dermatologische oder pharmazeutische Formulierungen enthaltend mindestens einen Emulgator gemäß mindestens einem der Ansprüche 1 bis 4.

10. Formulierung gemäß Anspruch 9, **dadurch gekennzeichnet, dass** die Formulierung eine Konzentration an Polyether enthaltenden Verbindungen kleiner 0,1 Gew.-% bezogen auf die Gesamtformulierung aufweist.

11. Kosmetische Formulierungen gemäß Anspruch 9 oder 10, die pigmenthaltig sind.

12. Gegebenenfalls dispergierte Feststoffe enthaltende Pflege- und Reinigungsmittel für Haushalt oder Industrie und Pflege- und Reinigungsmittel für harte Oberflächen, Leder oder Textilien enthaltend mindestens einen Emulgator gemäß mindestens einem der Ansprüche 1 bis 4.

## Claims

1. Emulsifier comprising at least one glycerin-modified siloxane of the general formula (I)
M_{2+f+2g-a-b} M' ₐ M" _{b} D_{c} D'_{d} D" ₑ T_{f} Q_{g} formula (I)
where
M = (R¹₃Si O_{1/2}),
M' = (R¹₂R² Si O_{1/2}),
M" = (R¹₂R³ Si O_{1/2}),
D = (R¹₂ Si O_{2/2}),
D' = (R¹R² Si O_{2/2}),
D" = (R¹R³ Si O_{2/2}),
T = (R¹ Si O_{3/2}) and
Q = (Si O_{4/2}),
where
R¹ = independently of the others, identical or different linear or branched, optionally aromatic hydrocarbon radicals having 1 to 16 carbon atoms, which optionally carry OH or ester functions,
R² = independently of the others, identical or different linear or branched alkyl radicals having 8 to 28 carbon atoms and
R³ = -CH₂CH₂CH₂OCH₂CH (OH) CH₂OH,
where
a = 0 to 2,
b = 0 to 2,
c = 20 to 300,
d = 4 to 130,
e = 3 to 75,
f = 0 to 10 and
g = 0 to 5
with the proviso that
N = 2 + c + d + e + 2f + 3g = 51 to 350,
y = (a + d)/(b + e) = 1 to 6.5 and
z = a + b + d + e = greater than 10.

2. Emulsifier according to Claim 1, **characterized in that**
2≥a+b>0.

3. Emulsifier according to Claim 1 or 2, **characterized in that** the glycerin-modified siloxanes comprise additional siloxane units of the general formula (II)
[D"'-R⁴-D"'] formula (II)
where
D"'= (R¹SiO_{2/2}),
where
R⁴ = -CH₂CHR¹-R⁵-CHR¹CH₂- or -CH=CR¹-R⁵-CR¹=CH- and
R⁵ = a linear or branched alkylene radical which optionally has ether, alcohol, ester, amine or siloxane groups, or a radical of the general formula -(Si(CH₃)₂O)ₕ-Si(CH₃)₂-, where h=1-400.

4. Emulsifier according to at least one of Claims 1 to 3, **characterized in that** mixtures of the glycerin-modified siloxanes with different Si chain lengths are present.

5. Use of at least one emulsifier according to at least one of Claims 1 to 4 for the preparation of water-in-oil or water-in-silicone emulsions, and for the preparation of cosmetic, dermatological or pharmaceutical formulations.

6. Use according to Claim 5 for the preparation of cosmetic emulsions which comprise inorganic pigments or cosmetic particles.

7. Use according to Claim 5 for the preparation of care and cleaning compositions for domestic use or industry, in particular for hard surfaces, leather or textiles, optionally comprising dispersed solids.

8. Water-in-oil, water-in-silicone emulsions and dispersions comprising at least one emulsifier according to at least one of Claims 1 to 4.

9. Cosmetic, dermatological or pharmaceutical formulations comprising at least one emulsifier according to at least one of Claims 1 to 4.

10. Formulation according to Claim 9, **characterized in that** the formulation has a concentration of compounds containing polyethers of less than 0.1% by weight based on the total formulation.

11. Cosmetic formulations according to Claim 9 or 10, which are pigment-containing.

12. Care and cleaning compositions for domestic use or industry and care and cleaning compositions for hard surfaces, leather or textiles, optionally comprising dispersed solids and comprising at least one emulsifier according to at least one of Claims 1 to 4.

## Revendications

1. Émulsifiant contenant au moins un siloxane modifié avec de la glycérine de formule générale (I)
M_{2+f+2g-a-b} M' ₐ M"_{b b} D_{c} D' _{d} D" ₑ T_{f} Q_{g} Formule (I)
avec
M = (R¹₃Si O_{1/2}),
M' = (R¹₂R² Si O_{1/2}),
M" = (R¹₂R³ Si O_{1/2}),
D = (R¹₂ Si O_{2/2}),
D' = (R¹R² Si O_{2/2}),
D" = (R¹R³ Si O_{2/2}),
T = (R¹ Si O_{3/2}) et
Q = (Si O_{4/2}),
avec
R¹ = indépendamment les uns des autres, radicaux hydrocarbonés identiques ou différents, linéaires ou ramifiés, éventuellement aromatiques, contenant 1 à 16 atomes de carbone, qui portent éventuellement des fonctions OH ou ester,
R² = indépendamment les uns des autres, radicaux alkyle identiques ou différents, linéaires ou ramifiés, contenant 8 à 28 atomes de carbone, et
R³ -CH₂CH₂CH₂OCH₂CH(OH)CH₂OH,
avec
a = 0 à 2,
b = 0 à 2,
c = 20 à 300,
d = 4 à 130,
e = 3 à 75,
f = 0 à 10 et
g = 0 à 5
à condition que
N = 2 + c + d + e + 2f + 3g = 51 à 350,
y = (a + d)/(b + e) = 1 à 6,5 et
z = a + b + d + e = supérieur 10.

2. Émulsifiant selon la revendication 1, **caractérisé en ce que** 2≥a+b>0.

3. Émulsifiant selon la revendication 1 ou 2, **caractérisé en ce que** les siloxanes modifiés avec de la glycérine contiennent des unités siloxane supplémentaires de formule générale (II)
[D"'-R⁴-D'"] Formule (II)
avec
D"' = (R¹SiO_{2/2}),
R⁴ = -CH₂CHR¹-R⁵-CHR¹CH₂- ou -CH=CR¹-R⁵-CR¹=CH- et
R⁵ = un radical alkylène linéaire ou ramifié, qui comprend éventuellement des groupes éther, alcool, ester, amine ou siloxane, ou un radical de formule générale - (Si(CH₃)₂O)ₕ-Si(CH₃)₂-, avec h=1-400.

4. Émulsifiant selon au moins l'une quelconque des revendications 1 à 3, **caractérisé en ce que** des mélanges des siloxanes modifiés avec de la glycérine de longueurs de chaîne Si différentes sont contenus.

5. Utilisation d'au moins un émulsifiant selon au moins l'une quelconque des revendications 1 à 4 pour la fabrication d'émulsions eau dans huile ou eau dans silicone, ainsi que pour la fabrication de formulations cosmétiques, dermatologiques ou pharmaceutiques.

6. Utilisation selon la revendication 5 pour la fabrication d'émulsions cosmétiques, qui contiennent des pigments inorganiques ou des particules cosmétiques.

7. Utilisation selon la revendication 5 pour la fabrication de produits de soin et de nettoyage contenant éventuellement des solides dispersés pour la maison ou l'industrie, notamment pour surfaces dures, cuir ou textiles.

8. Émulsions eau dans huile, eau dans silicone et dispersions contenant au moins un émulsifiant selon au moins l'une quelconque des revendications 1 à 4.

9. Formulations cosmétiques, dermatologiques ou pharmaceutiques contenant au moins un émulsifiant selon au moins l'une quelconque des revendications 1 à 4.

10. Formulation selon la revendication 9, **caractérisée en ce que** la formulation présente une concentration en composés contenant du polyéther inférieure à 0,1 % en poids, par rapport à l'ensemble de la formulation.

11. Formulations cosmétiques selon la revendication 9 ou 10, qui contiennent des pigments.

12. Produits de soin et de nettoyage contenant éventuellement des solides dispersés pour la maison ou l'industrie et produits de soin et de nettoyage pour surfaces dures, cuir ou textiles, contenant au moins un émulsifiant selon au moins l'une quelconque des revendications 1 à 4.
